(19) Europäisches Patentamt
European Patent Office
Office européen des brevets

(11) Veröffentlichungsnummer: **0 192 098 B1**

(12) # EUROPÄISCHE PATENTSCHRIFT

(45) Veröffentlichungstag der Patentschrift: **21.08.91**

(51) Int. Cl.$^5$: **A61K 31/55**

(21) Anmeldenummer: **86101249.0**

(22) Anmeldetag: **31.01.86**

(54) **Verwendung des 2-Amino-6-allyl-5,6,7,8-tetrahydro-4H-thiazolo[4,5-d]azepins zur Herstellung eines zur Behandlung der Parkinsonschen Erkrankung bzw. des Parkinsonismus geeigneten Arzneimittels.**

(30) Priorität: **06.02.85 DE 3503963**
**06.03.85 DE 3507861**

(43) Veröffentlichungstag der Anmeldung:
**27.08.86 Patentblatt 86/35**

(45) Bekanntmachung des Hinweises auf die Patenterteilung:
**21.08.91 Patentblatt 91/34**

(84) Benannte Vertragsstaaten:
**AT BE CH DE FR GB IT LI LU NL SE**

(56) Entgegenhaltungen:

**J. PHARM. PHARMACOL., Bd. 35, 03. Januar 1983, Seiten 543-545, M. GRABOWSKA-ANDEN et al.: "Stimulation of postsynaptic DA2receptors produces jerks in reserpine-treated rats"**

**FEDERATION PROCEEDINGS, Band 32, Nr. 2, Februar 1973, Seiten 183-190, O. HORNYKIEWICZ: "Parkinson's desease: from brain homogenate to treatment"**

(73) Patentinhaber: **BOEHRINGER INGELHEIM KG**
**Postfach 200**
**W-6507 Ingelheim am Rhein(DE)**
(84) Benannte Vertragsstaaten:
**BE CH DE FR IT LI LU NL SE AT**

Patentinhaber: **BOEHRINGER INGELHEIM INTERNATIONAL GmbH**
**Postfach 20**
**W-6507 Ingelheim am Rhein(DE)**
(84) Benannte Vertragsstaaten:
**GB**

(72) Erfinder: **Hornykiewicz, Oleh, Prof.Dr.**
**Esteplatz 7/10**
**A-1030 Wien(AT)**
Erfinder: **Hinzen, Dieter, Prof.Dr.**
**Konrad-Adenauer-Strasse 26**
**W-6501 Zornheim(DE)**
Erfinder: **Schingnitz, Günter, Dr.**
**Unter den Gärten**
**W-6550 Kreuznach 14(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 192 098 B1**

Rank Xerox (UK) Business Services

ACTA NEUROL. SCAND., Band 72, Nr. 6, 1986, Seiten 584-589, R.J. ANDERSON: "Modification of reserpine induced rigidity by dopaminergic and alpha-adrenergic drugs"

FORTSCHRITTE DER ARZNEIMITTELFORSCHUNG, Band 105, Nr. 5, 1987, Seiten 86-88, M. HERSCHEL: "Der Dopamin-Rezeptor; Angriffspunkt verschiedener Erkrankungen"

SELECTA, Band 46, 1982, Seiten 4344-48

## Beschreibung

In den belgischen Patentschriften 684 415 und 771 330 werden u.a. Thiazolo- und Oxazolo-Derivate der allgemeinen Formel I

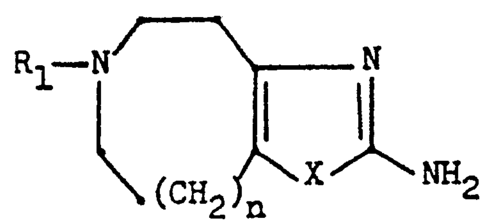

(I)

in der
$R_1$ ein Wasserstoffatom, eine gegebenenfalls durch eine Hydroxylgruppe substituierte Alkylgruppe mit 1 bis 4 Kohlenstoffatomen, eine gegebenenfalls durch ein Halogenatom, eine Methyl- oder Methoxygruppe substituierte Benzylgruppe oder eine Allylgruppe,
n die Zahl 2 oder auch 1, wenn X ein Schwefelatom darstellt, und
X ein Sauerstoff- oder Schwefelatom bedeutet, und deren physiologisch verträgliche Säureadditionssalze mit anorganischen oder organischen Säuren beschrieben.

Aus den belgischen Patentschriften 684 415 und 771 330 ist bekannt, daß die Verbindungen der allgemeinen Formel I und deren physiologisch verträgliche Säureadditionssalze wertvolle pharmakologische Eigenschaften aufweisen. So weisen die in der belgischen Patentschrift 684 415 beschriebenen Verbindungen insbesondere eine analgetische, sedative, antitussive, antipyretische und antiphlogistische Wirkung und die in der belgischen Patentschrift 771 330 beschriebenen Verbindungen je nach ihrer Substitution insbesondere eine blutdrucksenkende, sedative, hustenstillende und/oder antiphlogistische Wirkung auf. Desweiteren geht aus den obengenannten Patentschriften hervor, daß die Thiazolo-Derivate der allgemeinen Formel I, in der $R_1$ eine Alkylgruppe mit 1 bis 4 Kohlenstoffatomen oder eine Allylgruppe, n die Zahl 2 und X ein Schwefelatom bedeuten, insbesondere eine blutdrucksenkende Wirkung und die Oxazolo-Derivate der allgemeinen Formel I, in der $R_1$ ein Wasserstoffatom, eine gegebenenfalls durch eine Hydroxygruppe substituierte Alkylgruppe mit 1 bis 4 Kohlenstoffatomen oder eine Allylgruppe, n die Zahl 2 und X ein Sauerstoffatom bedeuten, insbesondere hustenstillende Eigenschaften aufweisen.

Es ist aus der EP-A1-0 005 732 bekannt, daß die Verbindungen der obigen allgemeinen Formel I, in der n die Zahl 2 darstellt, auch eine antanginöse Wirksamkeit aufweisen.

Ferner ist aus der US-Patent Schrift 4 400 378 bekannt, daß die Verbindungen der allgemeinen Formel I auch eine Antiglaucoma-Wirksamkeit aufweisen.

Gemäß den bisher vorliegenden wissenschaftlichen Veröffentlichungen wurde die Verbindung 2-Amino-6-allyl-5,6,7,8-tetrahydro-4H-thiazolo-[4,5-d]azepindihydrochlorid (B-HT 920) auch als ein selektiv auf präsynaptische dopaminerge Rezeptoren wirkender Agonist aufgefaßt [z.B. Andén et al., Acta Pharmacol. et Toxicol., 52, 51-56 (1983) und J. Neural Transmission 59, 129-137 (1983)].

Grundlage der Erfindung ist nun die überraschende Erkenntnis daß B-HT 920 an denervierten oder degenerierten postsynaptischen dopaminergen Strukturen des Gehirns ebenfalls eine agonistische Wirkung entfaltet. Diese Erkenntnis zeichnet die Verbindung im besonderen Maße zur Behandlung der Parkinsonschen Erkrankung bzw. des Parkinsonismus aus. Diese Erkrankungen werden durch eine Degeneration dopaminerger Neuronen im Gehirn verursacht. Der bei neuronaler Aktivität somit in nicht ausreichender Menge freigesetzte chemische Überträger Dopamin führt zum Auftreten der Parkinson-Symptomatik.

Nilsson & Carlsson (TIPS Reviews, Elsevier Biomedical Press, August 1982, S.322 ff.) erklären zur Behandlung des Parkinsonismus bzw. der Parkinsonschen Erkrankung solche Substanzen für die Vorteilhaftesten, welche eine spezifische agonistische Wirkung auf die postsynaptischen dopaminergen Neuren ausüben. Ferner schreiben Goodale et al., Science 210, 1141-1143 (1980) "...eine Verbindung, welche in erster Linie die postsynaptischen Rezeptoren aktiviert, wäre bei Krankheiten mit herabgesetzter Dopaminfreisetzung, wie etwa der Parkinsonschen Erkrankung, angezeigt".

Gegenstand der Erfindung ist daher die Verwendung des 2-Amino-6-allyl-5,6,7,8-tetrahydro-44-thiazolo-[4,5-d] azepins oder eines seiner Säureadditionssalze zur Herstellung für die Behandlung der Parkinsonschen-Erkrankung bzw. Parkinsonismus geeigneter Arzneimitteln.

Zur Behandlung der Parkinsonschen-Erkrankung bzw. Parkinsonismus lassen sich die Verbindungen A und ihre geeigneten Säureadditionssalze in die üblichen galenischen Zubereitungsformen für orale, parenterale, rektale oder transdermale Anwendung einarbeiten.

Die orale Einzeldosis beträgt am Menschen normalerweise zwischen 2,5 µg und 350 µg, vorzugsweise zwischen 100 µg und 250 µg; bei Mehrfachapplikation (z.B. 3 x täglich) beträgt die Tagesdosis bevorzugt zwischen 15 und 900 µg, vorzugsweise zwischen 30 und 750 µg. Die parenteralen und rektalen Dosen können in gleichem Bereich wie die oral anzuwendenden Substanzmengen liegen.

In der deutschen Patentameldung P 35 03 963.9 ist eine Untersuchung der Anti-Parkinson-Wirkung von B-HT 920 beschrieben. Die Untersuchung wurde an der Ratte durchgeführt.

Zur Untersuchung werden die folgenden Methoden verwendet.

(A) Spontan motorische Aktivität an der "naiven" Ratte

Registriert mit einem bekannten "Opto-Varimex 3" wurden zwei Aspekte der Motorik

a) "Ambulatorische" Bewegungen während der ersten 5 Minuten nach s.c. Injektion des B-HT 920; diese Phase der Lokomotorik entspricht der sogenannten "exploratorischen Aktivität" der Ratten in neuer Umgebung;

b) "nicht-ambulatorische" Bewegungen, die sich im wesentlichen aus stereotypen Bewegungen zusammensetzen, wobei mit ihrer Registrierung 45 Minuten nach Einbringen der Tiere in den Registrierkäfig (und 55 Minuten nach s.c. Injektion) begonnen wurde.

Testergebnis

Exploratorische Aktivität und stereotype Bewegungen

Es wurden folgende Dosen von B-HT 920 getestet: 0,02, 0,2, 2,0 mg/kg s.c.. Zum Vergleich wurde Apomorphin in der Dosis 0,1 mg/kg s.c. getestet.

Die nachfolgende Tabelle enthält die gefundenen Werte:

Tabelle 1

| Dosis mg/kg | Kontaktunterbrechungen (Exploratorische Aktivität /5 min.) | Stereotype Bewegungen Bewegungen /15 min. |
|---|---|---|
| B-HT 920 | | |
| 0 (Kontrolle) | 680 | 370 |
| 0,02 | 380 | n.t. |
| 0,2 | 150 | 370 |
| 2,0 | 130 | 310 |
| 4,0 | n.t. | 340 |
| Apomorphin | | |
| 0,1 | 150 | n.t. |
| 4,0 | n.t. | 650 |

n.t. = nicht getestet.

Die hemmende Wirkung von B-HT 920 auf die exploratorische Aktivität war demnach bereits fast maximal bei der Dosis von 0,2 mg/kg und war vergleichbar mit einem entsprechenden Effekt von 0,1 mg/kg Apomorphin (welches die Anzahl von Kontaktunterberechungen pro 5 Minuten ebenfalls auf 150 senkte).

Betr. stereotype Bewegungen, B-HT 920 führte in keiner der getesteten Dosen zur Erhöhung der Anzahl der stereotypen Bewegungen.

Im Gegensatz zu B-HT 920 führten 4,0 mg/kg Apomorphin zu einer Erhöhung der Anzahl der Stereotypen auf den Wert von 650.

(B) Ipsilaterale Rotation im Ibotensäure-Modell der Ratte

In Analogie zum entsprechenden Kainsäure-Modell nach Schwarcz et al.[Brain Res. 170 (1979) 485-495]. Dieses Modell beruht darauf, daß durch unilaterale intra-striatale (stereotaktische) Injektion des Neurotoxins, Ibotensäure diejenigen Striatumzellen, an denen u.a. auch post-synaptische Dopamine (DA) Rezeptoren sitzen, zerstört werden [vgl. Schwarcz et al., Exper. Brain Res. 37 (1979) 199-216], so daß post-synaptische DA Agonisten nur auf die entsprechenden DA-Rezeptoren der intakten Seite wirken können und daher Rotationsbewegungen des Tieres zur Seite des lädierten Striatums hervorrufen.

Testergebnis

Es wurden folgende Dosen von B-HT 920 getestet, 0,2, 0,5, 1,0 und 2,0 mg/kg s.c.. Zum Vergleich wurde Apomorphin in der Dosis 0,1 mg/kg
s.c. getestet. Die nachfolgende Tabelle enthält die gefundenen Werte:

TABELLE 2

| | Ipsilaterale Rotationen/Wirkungsdauer bis 3 Std. (Apomorphin) bis 6 Std. (B-HT 920) | | | | |
|---|---|---|---|---|---|
| Wirkstoff / Dosis mg/kg | 0,2 | 0,5 | 1,0 | 2,0 | 10,0 |
| B-HT 920 | 37 | 54 | 102 | 96 | - |
| Apomorphin | - | 265 | 436 | - | 880 |

Der Vergleich zwischen Apomorphin und B-HT 920 zeigt, daß B-HT 920 nur etwa 1/8 der post-synaptischen DA-agonistischen Wirkung des Apomorphins am normosensitiven DA-Rezeptor besitzt, d.h. in dieser Beziehung nur schwach wirksam ist.

(C) Kontralaterale Rotation am 6-OHDA-Modell- nach Ungestedt (Acta physiol scand, suppl 367 (1971) 69 ff; Europ. J. Pharmacol. 98 (1984) 165-176). Dieses Modell beruht darauf, daß durch stereotaktische unilaterale Injektion des Neurotoxins 6-OHDA die nigrostiatale DA Bahn einseitig zerstört wird, so daß die postsynaptischen DA-Rezeptoren im ipsilateralen Striatum ihrer DA Innervation beraubt, d.h. denerviert, werden und infolgedessen eine (Denervierungs-)Überempfindlichkeit entwickeln. An diesem Modell verursachen post-synaptisch wirksame DA-Agonisten bereits in niedereren Dosen durch preferentielle Wirkung auf die überempfindlichen DA Rezeptoren Rotationsbewegungen zur kontralateralen Seite.

TESTERGEBNIS

Es wurden folgende Dosen von B-HT 920 getestet: 0,02, 0,05, 0,5, 1,0 mg/kg. Zum Vergleich wurde

Apomorphin in der Dosis 0,05 mg/kg s.c. getestet. Die nachfolgende Tabelle enthält die gefundenen Werte:

| | Kontralaterale Rotationen bis 6 Stunden | |
|---|---|---|
| Dosis mg/kg | B-HT 920 | Apomorphin |
| 0,02 | 168 | - |
| 0,05 | 582 | 557 |
| 0,5 | 1282 | - |
| 1,0 | 1443 | - |

Im Vergleich zu B-HT 920 führten 0,05 mg/kg Apomorphin zu 557 kontralaterialen Rotationen/Wirkungsdauer. Es kann daher gefolgert werden, daß B-HT 920 im Gegensatz zu seiner schwachen Wirkung am normalen Da-Rezeptoren am denervierten Rezeptor ausgesprochen stark wirksam ist.

Die Entdeckung des Neurotoxins 1-Methyl-4-phenyl-1,2,3,6-tetrahydropyridin (MPTP) vor eineinhalb Jahren [Langston et al., Science 219, 979 (1983)] hat ein weiteres Tiermodell für die Parkinsonsche Erkrankung zur Verfügung gestellt.

Das durch MPTP beim Menschen und beim Affen ausgelöste, irreversible, neurologische Krankheitsbild ähnelt in seiner klinischen, pathologischen, biochemischen und pharmakologischen Ausprägung weitgehend der idiophathischen Parkinsonschen Erkrankung [Markey et al., Nature 311, 464 (1984)]. Ursache dieser überzeugenden Übereinstimmung ist die Tatsache, daß MPTP selektiv jene kleine Gruppen dopaminerger Nervenzellen in der Substantia nigra des Gehirns zerstört, welche auch bei der natürlich auftretenden Parkinsonschen Erkrankung durch degenerative Prozesse zerstört werden. Es wird so gar diskutiert, daß auch die Ursache der idopathischen Parkinsonschen Erkrankung im Organismus entstehendes MPTP oder eine ähnliche chemische Verbindung ist [Snyder, S.H., Nature 311, 514 (1984)]. Möglicherweise bedingt durch den spezifischen Metabolismus des MPTP ist die klinische Ausprägung des MPTP-Parkinsonbildes bisher außer beim Menschen nur noch beim Affen nachweisbar.

Das am Rhesusaffen verwirklichte MPTP-Modell ist daher in hervorragendem Maße geeignet, die Wirkung von Anti-Parkinson-Medikamenten zu prüfen. 7 Rhesusaffen wurden mit MPTP (3 Tage lang 1 x 0.15 mg/kg i.m. täglich, 3 Tage Pause, weitere 3 Tage 1 x 0,30 - 0,40 mg/kg täglich) appliziert und wiesen folgende Symptome auf: die Tiere waren akinetisch und nicht in der Lage, Wasser und Futter aufzunehmen. Sie zeigten eine typische gebeugte Haltung: gelegentlich treten kataleptische Zustände auf. Die Extremitäten wiesen einen Rigor auf, welcher bei passiver Bewegung von klonischen Krampfen durchbrochen wurde. Willkürbewegungen des Rumpfes und der Extremitäten waren in der Regel auch durch stärkste, schmerzhafte Reize nicht auszulösen.

Nach der intramuskulären Gabe von B-HT 920 (50-100 $\mu$g/kg) traten im zeitlichen Abstand von 5 bis 10 min erste Willkürbewegungen auf, die in den folgenden 10 bis 30 min von einer allmählichen, weitestgehenden Normalisierung der Motorik gefolgt war. Die Tiere waren in der Lage, Nahrung aufzunehmen. Sie verhielten sich innerhalb ihrer Käfige regelrecht, dies galt auch hinsichtlich Vigilanz und artspezifischen Verhaltens. Als Restsymptomatik wurden gelegentlich vorübergehender, leichter Ruhetremor und Verringerung der groben Kraft registriert. Eine Sedation trat nicht ein. Die Hautdurchblutung erschien gegenüber

dem Zustand vor Gabe von B-HT 920 gesteigert.

Die Wirkung von B-HT 920 ließ nach ca. 3 Stunden nach, und die Tiere verfielen wieder in die oben beschriebene parkinson-Symptomatik; eine erneute Applikation von B-HT 920 führte wieder zur Besserung bzw. weitgehenden Aufhebung der klinisch pathologischen Erscheinung. Die vorteilhafte Wirkung von B-HT 920 wurde an jedem einzelnen Tier somit mehrfach reproduziert.

Nebenwirkungen traten in den bisher angewandten Dosierungen nicht in Erscheinung.

Die Verwendung der Verbindung und deren Säureadditionssalze hat den Vorteil vor den z.Z. üblichen DA-Agonisten, daß diese Substanz nur auf die denervierten post-synaptischen Gehirn-DA-Rezeptoren einwirken würden, so daß sich beim Parkinson-Patienten die pharmakologische (motorische) Wirkung von B-HT 920 vor allem auf jene Gehirn-DA-Systeme beschränken würde, die von der Erkrankung betroffen sind. Mit anderen Worten:
die Verbindung und ihre Säureadditionssalze wirken besonders stark bei schweren Parkinson-Fällen (hoher Denervierungsgrad der Striatum-DA-Rezeptoren).

Des weiteren weist die Verbindung und ihre physiologisch verträglichen Säureadditionssalze eine gute Verträglichkeit auf, beispielsweise beträgt die orale $LD_{50}$ an der MAUS 455 mg/kg.

Beispiel 1

Dragéekern

Zusammensetzung:

| 1 Dragéekern enthält: | |
|---|---|
| B-HT 920 | 50 µg |
| Milchzucker | 38,45 mg |
| Maisstärke | 10,0 mg |
| Gelatine | 1,0 mg |
| Magnesiumstearat | 0,5 mg |
| | 50,0 mg |

Herstellungsverfahren:

Die Mischung der Wirksubstanz mit Milchzucker und Maissärke wird mit einer 10 %igen wäßrigen Gelatinelösung durch Sieb 1 mm granuliert, bei 40 °C getrocknet und nochmals durch obiges Sieb gerieben. Das so erhaltene Granulat wird mit Magnesiumstearat gemischt und zu Dragéekernen verpreßt. Die Herstellung muß in abgedunkelten Räumen vorgenommen werden.

Kerngewicht: 50 mg
Stempel: 5 mm, gewölbt

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 100 mg.

Beispiel II

Suppositorien

| | |
|---|---|
| 1 Zäpfchen enthält: | |
| B-HT 920 | 100,0 µg |
| Zäpfchenmasse (z.B. Witepsol W 45) | 1690,0 mg |

Herstellungsverfahren:

Die feingepulverte Substanz wird mit Hilfe eines Eintauchhomogenisators in die geschmolzene und auf 40° C abgekühlte Zäpfchenmasse eingerührt. Die Masse wird bei 35° C in leicht vorgekühlte Formen ausgegossen.

Beispiel III
Ampullen mit 200 µg B-HT 920

| | |
|---|---|
| 1 Ampulle enthält: | |
| B-HT 920 | 200 µg |
| Citronensäure | 7,0 mg |
| Natriumphosphat sek. .2 $H_2O$ | 3,0 mg |
| Natriumpyrosulfit | 1,0 mg |
| Dest. Wasser | ad 1,0 ml |

Herstellungsverfahren:

In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert pyrogenfrei.

Abfüllung: in braune Ampullen unter Schutzbegasung
Sterilisation: 20 Minuten bei 120° C

Die Herstellung und Abfüllung der Ampullenlösung muß in abgedunkelten Räumen vorgenommen werden.

Beispiel IV
Dragées mit 1 mg B-HT 920

| | |
|---|---|
| 1 Dragéekern enthält: | |
| B-HT 920 | 100 µg |
| Milchzucker | 36,0 mg |
| Maisstärke | 12,4 mg |
| Gelatine | 1,0 mg |
| Magnesiumstearat | 0,5 mg |
| | 50,0 mg |

Herstellungsverfahren:

Analog Beispiel I.

Kerngewicht: 50 mg

Stempel: 5 mm, gewölbt

Drageegewicht: 100 mg

Beispiel V

Dragées mit 0,2 mg B-HT 920

| 1 Drageekern enthält: | |
|---|---|
| B-HT 920 | 0,2 mg |
| Digoxin | 0,25 mg |
| Milchzucker | 66,55 mg |
| Kartoffelstärke | 25,0 mg |
| Polyvinylpyrrolidon | 2,0 mg |
| Magnesiumstearat | 1,0 mg |
| | 120,0 mg |

Herstellungsverfahren:

Die intensive Mischung der Wirksubstanzen mit Milchzucker und Kartoffelstärke wird mit einer 10 %igen Lösung des Polyvinylpyrrolidons in Äthanol durch Sieb 1,5 mm granuliert, bei 40° C getrocknet und nochmals durch Sieb 1,0 mm gerieben. Das so erhaltene Granulat wird mit Magnesiumstearat gemischt und zu Drageekernen verpreßt.

Kerngewicht: 120 mg

Stempel: 7 mm, gewölbt

Die so hergestellten Drageekerne werden nach bekanntem Verfahren mit einer Hülle überzogen, die im wesentlichen aus Zucker und Talkum besteht. Die fertigen Dragées werden mit Hilfe von Bienenwachs poliert.

Drageegewicht: 200 mg.

Beispiel VI

Gelatine-Steckkapseln mit 300 $\mu$g B-HT 920

1 Kapsel enthält:

| | |
|---|---|
| B-HT 920 | 0,3 mg |
| Codeinphosphat | 10,0 mg |
| Weinsäure | 3,0 mg |
| Maisstärke | 85,7 mg |
| | 100,0 mg |

Herstellungsverfahren:

Die Substanzen werden intensiv gemischt und in Opak-Kapseln geeigneter Größe abgefüllt.
Kapselfüllung: 100 mg

## Patentansprüche

1. Verwendung des 2-Amino-6-allyl-5,6,7,8-tetrahydro-4H-thiazolo[4,5-d]azepins (B-HT 920) oder eines seiner Säureadditionssalze zur Herstellung eines zur Behandlung der Parkinsonschen-Erkrankung bzw. Parkinsonismus geeigneten Arzneimittels.

## Claims

1. Use of 2-amino-6-allyl-5,6,7,8-tetrahydro-4H-thiazolo[4,5-d]azepine (B-HT 920) or of one of the acid addition salts thereof for preparing a pharmaceutical composition suitable for the treatment of Parkinson's disease or Parkinsonism.

## Revendications

1. Utilisation de la 2-amino-6-allyl-5,6,7,8-tétrahydro-4H-thiazolo[4,5-d]azépine (B-HT 920) ou de l'un de ses sels d'addition d'acide pour la préparation d'un médicament convenant au traitement de la maladie de Parkinson ou du parkinsonisme.